# EUROPEAN PATENT SPECIFICATION

(11) **EP 2 242 460 B1**
(45) Date of publication and mention of the grant of the patent: **09.11.2011**
(21) Application number: 09746301.2
(22) Date of filing: 14.05.2009
(51) Int. Cl.: A61F 5/00

(54) **ANKLE FOOT ORTHOSIS FOR COUNTERACTING THE SUPINATION/PRONATION OF THE FOOT**
FUSSGELENKORTHESE ZUR UNTERDRÜCKUNG DER AUFWÄRTS-/EINWÄRTSDREHUNG DES FUSSES
ORTHÈSE DE LA CHEVILLE DESTINÉE À CONTRER LA SUPINATION/PRONATION DU PIED

(30) Priority: 15.05.2008 IT VI20080110; 22.04.2009 IT VI20090087
(43) Date of publication of application: 27.10.2010
(73) Proprietor: Dolcetta, Diego, 20123 Milano (IT)
(72) Inventor: Dolcetta, Diego, 20123 Milano (IT)
(74) Representative: Vittorangeli, Lucia
(86) International application number: PCT/IT2009/000214
(87) International publication number: WO 2009/139019

(56) References cited:
- EP-A- 0 567 783
- EP-A- 1 114 626
- WO-A-2005/034819
- US-A- 3 680 549
- US-A- 5 219 324
- US-A1- 2005 234 378

## Description

The present invention relates to an ankle foot orthosis for counteracting the spasticity in varus-supination of the foot. In less common cases, the mirror embodiment can limit the deformation in valgus-pronation.

As is known, patients suffering from a motor deficiency of the lower limb, in order to compensate for the impaired motor functions, very frequently make use of orthoses. These are medical devices able to maintain passively given articular postures, in order to improve a function or support a part of the body.

More specifically, when the disorder affects the ankle and the foot, orthoses are used, these being referred to by the acronym AFO (Ankle Foot Orthosis) and consisting of orthopaedic supports which are applied externally in order to correct the pathological postures of the foot and improve the gait.

Many of the AFOs which are commercially available are based on an orthosis which is commonly known as a "Codivilla spring" originally conceived at the beginning of the past century, as a solution for the "foot drop" of peripheral origin and essentially composed of an L-shaped bar which may be fixed behind the leg and supporting the bottom of the foot so as to keep it correctly raised. It was called "spring" because it was able to return elastically the flexural force imparted to it by flexing of the ankle during the terminal part of the stance phase of step. The foot drop was quite a frequent condition at the time when Codivilla spring was invented, on the first half of the past century, due to peripheral nerves lesions, from work or war traumas.

During the second half of the past century, the clinic of the foot drop has been progressively modifying: the foot drop became rarely due to peripheral nerves lesions. When due to peripheral nerves lesions, these nowadays are seldom due to traumatic causes, but more often due to peripheral neuropathy, such as the diabetic one. Moreover, the whole foot drop problem is less frequently caused by peripheral nerves impairment. The prevalent cause of foot drop is currently a consequence of a central nervous system lesion.

A very common cause of foot drop of such "central" lesion is the hemiparesis following stroke.

The clinical features of the foot drop of "periferal" origin are very different from those of the foot drop of "central" origin. The former is characterized by simple loss of muscle strength and flaccidity, and final consequent atrophy. This is the foot drop type best benefiting of Codivilla spring. The latter, instead, despite sharing the "loss of function", and partially of strength, can be distinguished by the presence of a complex pattern of pathological reflexes characterized by spasticity. It is not generally present while the lower limb is at rest, but eminently appears during the gait: the damaged brain find impossible to correctly control the movement, even if peripheral nerves are undamaged. In particular, hemiparetics suffer from a motor deficiency of the lower limb which, while walking, is manifested in the form of spasticity of varying severity, with stiffness of the paretic limb, tendency to the knee hyperextension, the foot drop and the associated pathological reflexes: supination or inversion, namely the tendency for the foot to rotate about its antero-posterior axis turning the sole inwards during the whole step.

Starting from the Codivilla spring, many attempts has been made for adapting AFOs to clinical features of the "new" foot drop. They all bear the heavy limit of facing the problem only on the very same plane of Codivilla spring: the anteroposterior plane. They can outwordly remembering a Codivilla spring or, on the contrary, to look completely different. However, they all consider the main forces to be counteracted those acting on the anteroposterior plane: the foot drop and the knee hyperextension.

A technologically refined solution, which is commercially known by the name of "ToeOFF®", available on the market in many different versions, has a shaped bar, extending from the side portion of a semi-rigid sole-piece, and provided with a front leg grip which is aligned with the tibia and is fixed to the leg by means of a pair of Velcro® fasteners. Its main plane of action is the anteroposterior (or sagittal) one, providing a bound to the foot drop.

A much less charming embodiment counteracting the foot drop with an anterior approach is the more recent US-7458950. It also efficaciously avoids the foot drop. Unlike Codivilla spring, it completely misses its forward thrust due to the elastic return.

The fact that "ToeOFF®" reaches the footplate descending laterally to the ankle seems to make of it an aid for the foot drop of neurological origin, but its efficacy in counteracting the supination is negligible. The producers themselves, of that AFO and all its variants, declare its contraindication if the supination reflex is moderate or severe. However, unlike US-7458950, the forward thrust is perfectly maintained, and likely improved with respect to Codivilla spring. This is mainly due to the new materials used for the plantar realization.

Therefore, both US-7458950 and "ToeOFF®" work on the anteroposterior plane, approaching the leg from the front, basically binding the foot weight to the leg.

Despite its original appearance, the frontal AFOs which are at present commercially available, such as TOEOFF® and similar models made by other manufacturers (es. WO-02083040 and US-2005234378), are in fact designed to correct the "foot drop" and effectively oppose the supinatory movement only if the supination is of a very slight nature.

WO-2005034819, which is not commercially available, discloses an orthosis having a unitary body with a generally spiral shaped lower leg portion and a footplate portion. The orthosis is configured to allow simultaneous control of the knee and ankle-foot complex during locomotion.

The orthosis disclosed by WO-2005034819 is conceived to transfer mechanical energy when worn by a walking subject. It stops the knee from hyper-extending during the stance phase, by generating a knee flexion moment or torque. However, the orthosis of WO-2005034819 is not capable of counteracting the spasticity in varus-supination of the foot because its structure allows forces to operate on a rear-front longitudinal plane. In fact, this aim is not even mentioned In the patent by the inventors.

One of these AFOs, which is commercially known by the name of "DYNA ANKLE" and wich is disclosed by EP-0567783, comprises, for example, a kind of splint that surrounds at the rear the leg and the ankle to which it is fixed using straps. Despite remembering in shape the traditional Codivilla spring, the straps addition allows to quite efficaciously counteract the supinating movement.

Such type of orthosis is particularly difficult to fit, also considering that the person who wears it very often has the use of only one hand.

Other AFOs instead consist of a sole-piece, which has, extending from it medially or laterally, a substantially straight bar which terminates in a respective medial or lateral leg grip. Alternatively, a pair of haff-shells, one medial and the other lateral, may extend from the sole-piece, these extending beyond the tibio-tarsal articulation and retaining the foot with a Velcro® fastener, as in the orthosis which is commercially known by the name of "PEROMED".

In facts, none of the above-described structures are able to directly oppose the powerful inversion thrust exerted by the foot, but only limit it indirectly by means of semi-rigid fixing straps or collars, or by the anterior way of reaching the plantar.

The absence of AFOs specifically counteracting the supination has led, during the last decade, to the development of specific surgical techniques, such as functional tendon-transfer surgery, where the muscles that often participate to supination reflex (e.g. extensor hallucis longus) are deviated so as to acquire the opposite pronatory function.

Also, attempts have made to deactivate the muscle chiefly responsible for supination by means of paralysis induced by botulinic toxin infiltration.

The above method has a limited duration, 4 to 6 months, and in some cases may become less effective after a certain number of applications, owing to a gradual immunity to the toxin.

Both methods, in any case, often do not solve the problem because, where muscles responsible for supination are different than extensor hallucis longus, deviation or deactivation thereof may worsen the foot drop, ankle stability and gait.

All the conventional AFOs require tight shoes in order to be even slightly effective against supination and therefore cannot work properly with sandals, moccasins, open shoes and summer or light shoes in general.

Moreover, it must be considered that the AFOs, which are at present commercially available, are bulky, uncomfortable and generally not liked by patients.

The conventional AFOs are also always incompatible with the inner soles present in shoes - the plantar is flat, unlike the inner soles -, with the result that said soles must be removed.

In any case, the inner soles must be removed/substituted in order to equalize the legs height but the removal of the inner soles is often difficult, because they can be glued.

The conventional AFOs also make it necessary to always use a shoehorn when wearing the shoes because the rigid heel, made of plastics or carbon fiber, easily grazes the fingers when attempting to wear the shoe without a shoehorn.

None of the commercially available orthoses compatible with normal shoes are designed to counteract the supinatory movement of the foot, namely to provide a constraint physically opposing the supinatory lever action. This would find an application both in the aforementioned neurological patients suffering from paresis of central origin and in the orthopedic patients described below.

A sudden, excessive, unwanted supinatory movement of the foot, in the absence of neurological damages, can lead to traumatic, orthopedic consequences.

The ankle twisting is a frequent occurrence in young sportsmen. This often leads to recurrent ankle sprain and instep injures during the run. The ankle instability most often consists in a weakness of ligamentous structures limiting the supinatory movement.

The aim of the invention is to overcome the problems of the prior art devices, by providing an orthosis structure, in particular for the ankle/foot, which is truly effective in counteracting the deformities associated with varus-supination of the foot or the less common valgus-pronation.

In connection with the above mentioned aim, one particular object of the invention is to provide an orthosis structure which is able to provide greater stability of the ankle and foot and at the same time ensure a more dynamic and functional gait.

A further object of the invention is to provide an orthosis structure, which is able to control foot drop during the swinging phase.

A further object of the invention is to provide an orthosis structure, which can be easily and rapidly fitted with one hand only.

A further object of the invention is to provide an orthosis structure, which, in addition to being aesthetically pleasing, is light and comfortable.

A particular aim of the present invention is to provide an orthosis structure, which is mainly designed to oppose the supination movement of the foot.

A particular object of the invention is to provide an orthosis structure, which is able to provide a rigid constraint opposing the supinatory thrust and to support the foot, offering maximum rigidity in the frontal plane.

A further object of the present invention is to provide an orthosis structure, which favours the correct dynamics of the gait owing to its elasticity in the sagittal plane.

Another object of the present invention is to provide an orthosis structure which helps preventing the hyperextension of the knee.

Yet another object of the present invention is to provide an orthosis structure which may be useful not only for neurological patients - in order to counteract the supinatory reflex which is frequently observed in pareses due to central neurological lesion - but also for orthopaedic patients as a means for preventing recurrent twisting of the ankle (ankle sprain) and instep injuries.

A further object of the present invention is to provide an orthosis structure, which may be used with all commercially available footwear, of the closed or open type, including sandals, without having to modify the footwear after purchase.

Yet another object of the present invention is to provide an orthosis structure, which is able to oppose also supinatory forces much greater than the supinatory reflex characteristic of central paresis, such that it may also be used by orthopaedic patients such as those practising sporting activities.

A further object of the present invention is to provide an orthosis structure, which is able to return elastically the flexural force imparted to it by flexing of the ankle during the walking movement.

An object of the present invention is to provide an orthosis structure which, being slim and lightweight, is easy and convenient to use as well as being cooler during the summer months and aesthetically more pleasing in appearance, thereby reducing the embarrassment associated with wearing it together with sandals and shorts or skirts.

The above mentioned aim, as well as the objects mentioned and other objects which will become clear below, are achieved by an ankie foot orthosis for counteracting the supination of the foot, according to claim 1.

Further characteristic features and advantages will emerge more clearly from the description of preferred, but non-exclusive, embodiments of an orthosis structure according to the invention shown by way of a non-limiting example in the accompanying drawings in which:
Figure 1 is a front view and a perspective view of a leg and a foot affected by inversion;
Figure 2 is a front view of an orthosis structure according to the invention fitted onto a leg and a foot affected by inversion;
Figure 3 is a perspective view of the orthosis structure according to the invention fitted onto a leg and a foot affected by inversion;
Figure 4 is a side view of the orthosis structure according to the invention;
Figure 5 is a side view, opposite to the preceding one, of the orthosis structure according to the invention;
Figure 6 is a front view of a further embodiment of the orthosis structure according to the invention fitted onto a leg and a foot affected by inversion;
Figure 7 is a perspective view of the orthosis structure shown in the previous figure, fitted onto a leg and a foot affected by inversion;
Figure 8 is a perspective view of still a further embodiment of the orthosis structure according to the invention fitted onto a leg and a foot affected by inversion;
Figure 9 is a front view of a leg and a foot affected by inversion;
Figure 10 is a front view of a further embodiment of the orthosis structure according to the invention fitted onto a leg and a foot affected by inversion;
Figure 11 is a perspective view of the orthosis structure of the preceding figure;
Figure 12 is a perspective view of the orthosis structure of the preceding figure fitted onto a leg and a foot affected by inversion;
Figure 13 is a perspective view of a further embodiment of the orthosis structure, fitted onto a leg and a foot affected by inversion.

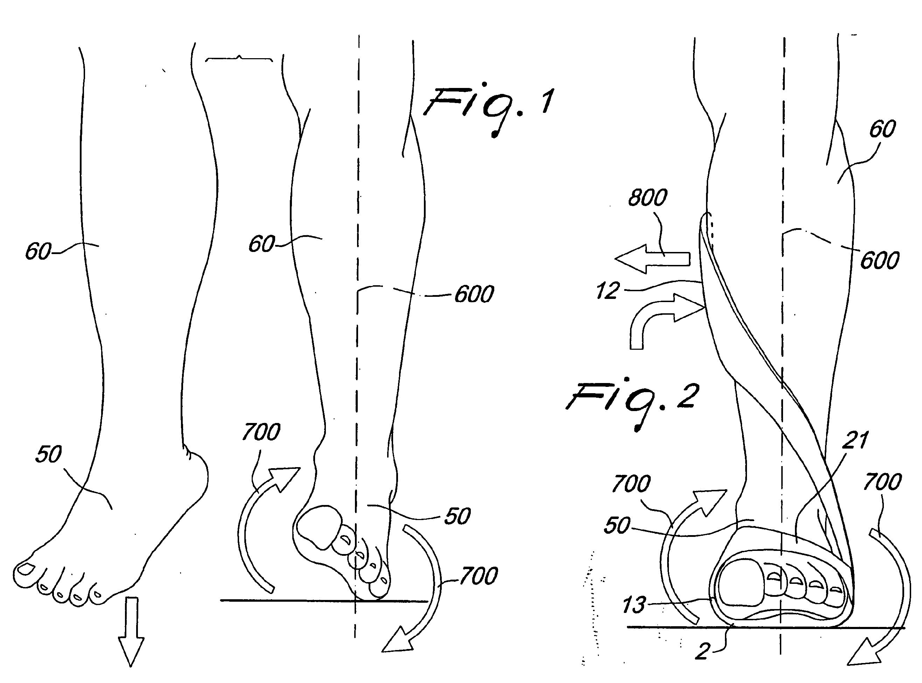

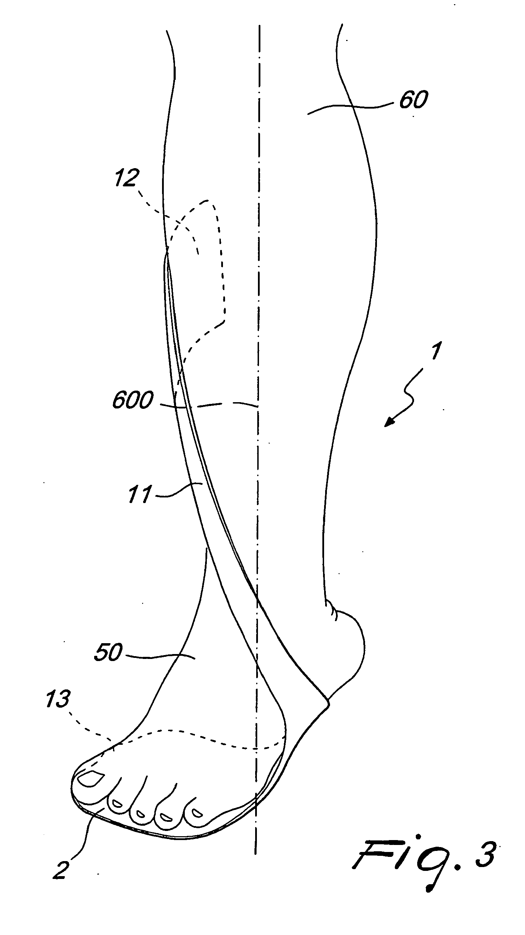

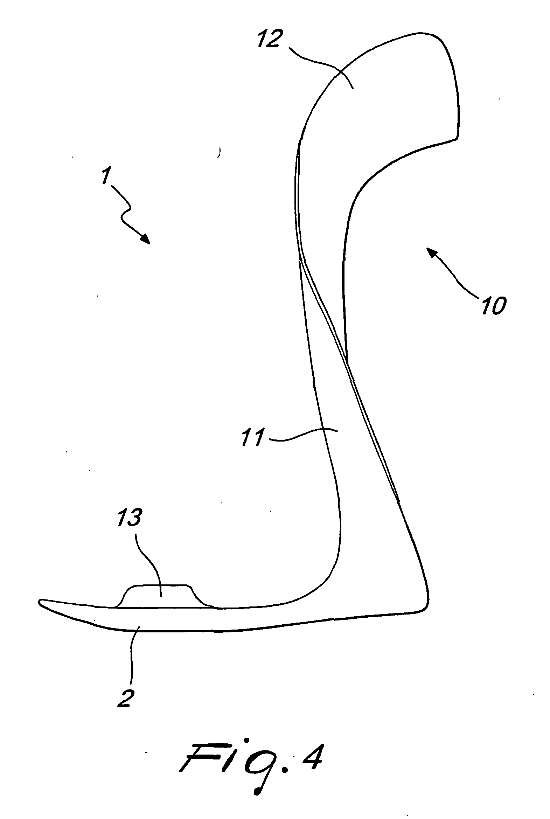

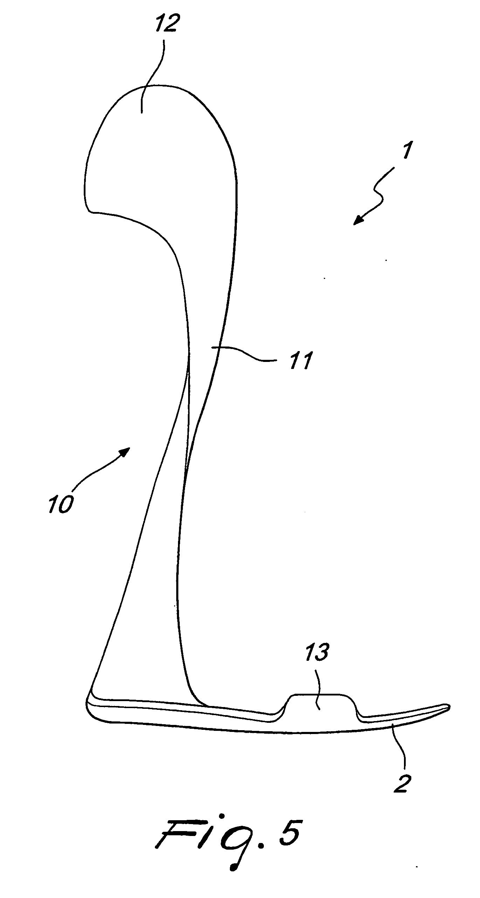

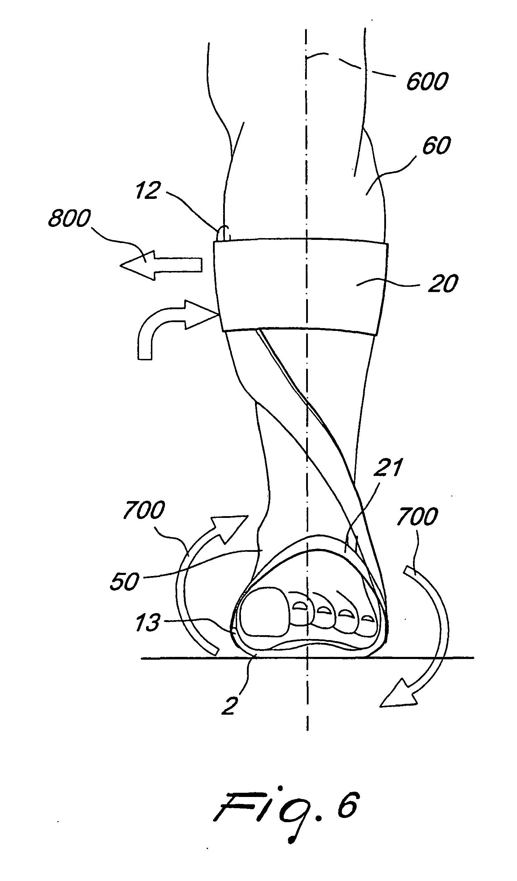

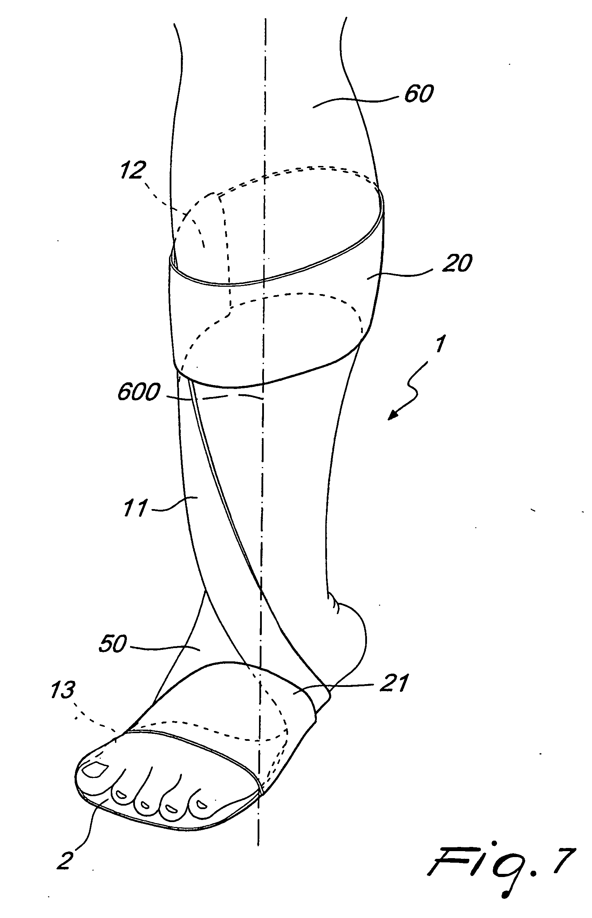

With reference to figures 1 - 8, the orthosis structure, according to the invention, globally designated by reference number 1, comprises a sole-piece 2 on which a foot 50 rests.

The sole-piece 2 is modelled so as to reproduce a portion of the sole of the foot 50 and, in particular, so as to support the forefoot. The sole-piece has a flexible structure, which, during the walking movement, allows an elastic return of the force applied to flex it. This feature provides the orthosis structure 1 with a dynamic characteristic, which allows a more gradual transfer between the putting-down phase and the pushing-off phase, facilitating the forward movement.

The sole-piece 2 has an anatomical cushioning insole (not shown in the accompanying figures), which is made of breathable material and designed so as to support the foot 50 at the physiologically correct points.

According to the invention, the orthosis structure 1 comprises medial/lateral gripping means 10 associated with the sole-piece 2 and that can be engaged respectively with the medial zone, or lateral zone, of the leg 60 with which the foot 50 is articulated.

A special feature of the medial/lateral gripping means 10 consists in the fact that the point where they are joined to the sole-piece 2, which corresponds essentially to the point where the torsional thrust 700 exerted by the foot 50 is applied, and their portion which can be engaged with the medial zone or lateral zone of the leg 60, which corresponds essentially to the point of application of the reaction of the constraint 800, are situated on opposite sides to each other relative to the longitudinal axis 600 of the leg 60.

According to a first embodiment of the invention, the orthosis structure 1 is designed to counteract the problem of varus-supination of the foot, also known as inversion, which is particularly frequent in patients affected by spasticity of the lower limbs. In this embodiment, the medial/lateral gripping means 10 essentially comprises a shaped lever 11 which is formed with the lateral - or outer - portion of the sole-piece 2 and is engaged with the medial - or inner - zone of the leg 60.

The sole-piece 2 is structured so as to engage with the part of the sole of the foot 50 corresponding to the forefoot and to the side (o lateral?) portion of the midfoot. These zones in fact have a fundamental role in opposing inversion of the foot 50 and supporting the latter.

The shaped lever 11, together with the sole-piece 2, is made of materials which make it at the same time lighter, very rigid and stronger, such as carbon fibre or Kevlar, and owing to its characteristics it is able to oppose actively the inversion thrust exerted by the foot 50, transmitting it rigidly to the medial zone of the leg 60. The inversion thrust of the foot 50 would in fact tend to rotate the shaped lever 11 in the medio-lateral direction, but the medial zone of the leg 60 provides a physical constraint opposing this rotation.

The sole-piece 2 is also counteracting the tendency of the medial portion of the foot to raise. Such tendency is counteracted by a restrain means associated with the sole-piece and preferably constituted by a strap 21 removably associated with the sole-piece 2.

The shaped lever 11 extends from the sole-piece 2 substantially along the portion associated with the midfoot, and extends so as to pass over the front pre-tibial zone of the leg 60, without necessarily making contact therewith. The shaped lever 11 terminates in a plate 12, which allows it to rest against the medial zone of the leg 60.

During manufacture, the length of the shaped lever 11 may be adjusted according to specific requirements.

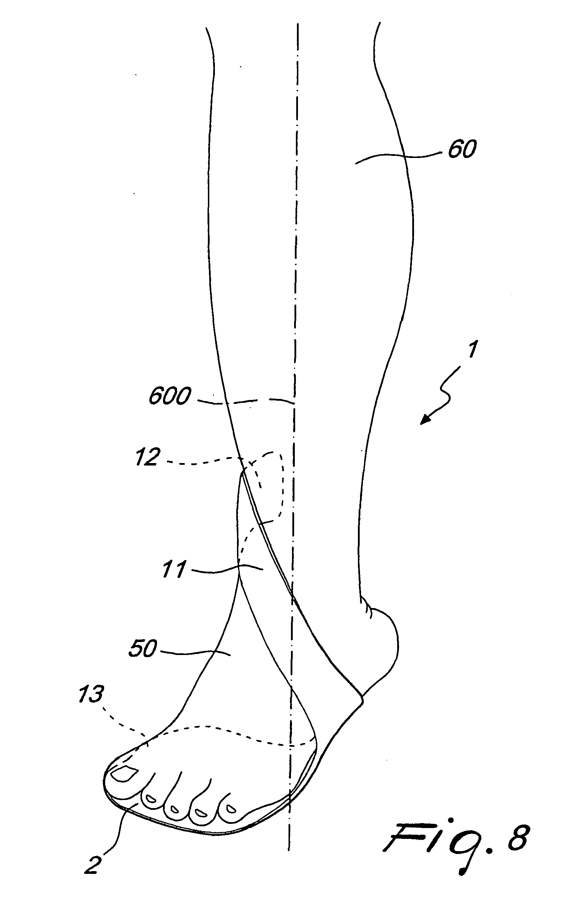

Namely, the length of the shaped lever 11 may vary from a minimum length, where the plate 12 rests against the medial zone of the leg 60 just above the tibial malleolus, as substantially illustrated in Figure 8, to a maximum length, where the plate 12 rests against the medial zone of the leg 60 just below the knee, as substantially illustrated in the Figures 2 to 7.

The shaped lever 11 is preferably inclined forwards at an angle variable substantially between 5° and 15° relative to the perpendicular of the sole-piece 2, in order to counteract hyperextension of the knee during the stance phase.

The shaped lever 11 is also provided with an anatomical padding (not shown in the accompanying figures) which lines it internally, improving the comfort during use. This padding is significantly thinner in the pre-tibial region of the leg 60, so as to prevent contact thereof with the shaped lever 11.

If necessary, the rigidity of the shaped lever 11 may be increased by means of a rigid core (not visible in the figures) preferably consisting of a metallic insert, which is suitably modelled and incorporated in the structure of the shaped lever 11 so as to engage inside the sole-piece 2.

The plate 12 may be supplemented with an adjustable band 20, which embraces the leg 60. The fact that the adjustable band 20 is joined to the plate 12, which already itself embraces a small portion of the leg 60, facilitates considerably fastening thereof. Owing to this feature, in fact, hemiparetic patients are also able to fit and fix the orthosis structure 1 easily and by themselves.

In order to further increase the effectiveness of the orthosis structure 1, a flange 13 projects from the medial portion of the sole-piece 2.

The flange 13 has the end of the strap 21 removably joined thereto. The strap 21 crosses over the dorsal portion of the foot 50 and has an opposite end removably joined to the shaped lever 11, at the point where it is joined to the sole-piece 2, or in a higher position on the lever itself. As a result of such a feature, together with the presence of a non-slip sole associated underneath the sole-piece 2 (not shown in the figures), the orthosis structure 1 can be used without necessarily wearing shoes.

The orthosis structure 1 may be completed by a stabilization appendage (not shown in the figures), which extends from the sole-piece 2 towards the hindfoot. The function of the stabilization appendage is that of counteracting extrarotation of the foot 50 during the putting-down phase.

Duly modified, the orthosis structure according to the invention may also be used to counteract valgus-pronation of the foot, also known as eversion, even though this problem is rarer from a clinical point of view.

In fact, according to a further embodiment of the invention (not shown in the accompanying figures), the orthosis structure has a shaped lever, which forms the medial/lateral gripping means, integral with the medial - or inner - portion of the sole-piece 2 and engaged with the lateral - or outer - zone of the leg 60.

The shaped lever, which is now designed to transmit rigidly the eversion thrust exerted by the foot 50 to the lateral zone of the leg 60 which acts as a constraint, extends from the sole-piece 2 substantially in the zone corresponding to the midfoot, and extends so as to pass over the front region of the leg 60. However, the shaped lever terminates in a plate, which allows it to rest against the side zone of the leg 60.

In this confguration the flange extends from the side portion of the sole-piece 2.

The operating principle of the orthosis structure according to the invention is described below.

The orthosis structure 1 is fitted by inserting the sole-piece 2 inside a shoe and resting the sole of the foot 50 on top of it.

The shaped lever 11 is arranged so as to pass over the tibial zone of the leg 60, while the plate 12 is rested against it in the medial or lateral zone thereof.

Such construction physically provides a constraint which opposes the rotational movements induced by inversion of the foot 50.

Then the strap 21 is fastened as well as the adjustable band 20, if present.

The above description clearly shows how, in each of the examples considered, the thrusts exerted by the foot 50 are actively counteracted by the medial/lateral gripping means 10 which form an integral part of the orthosis structure 1.

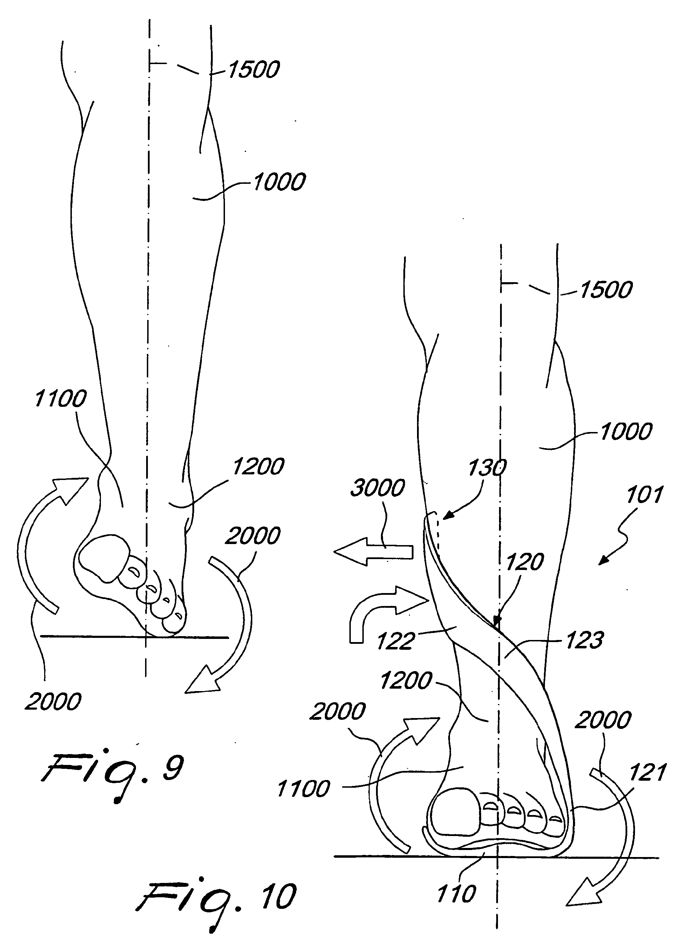

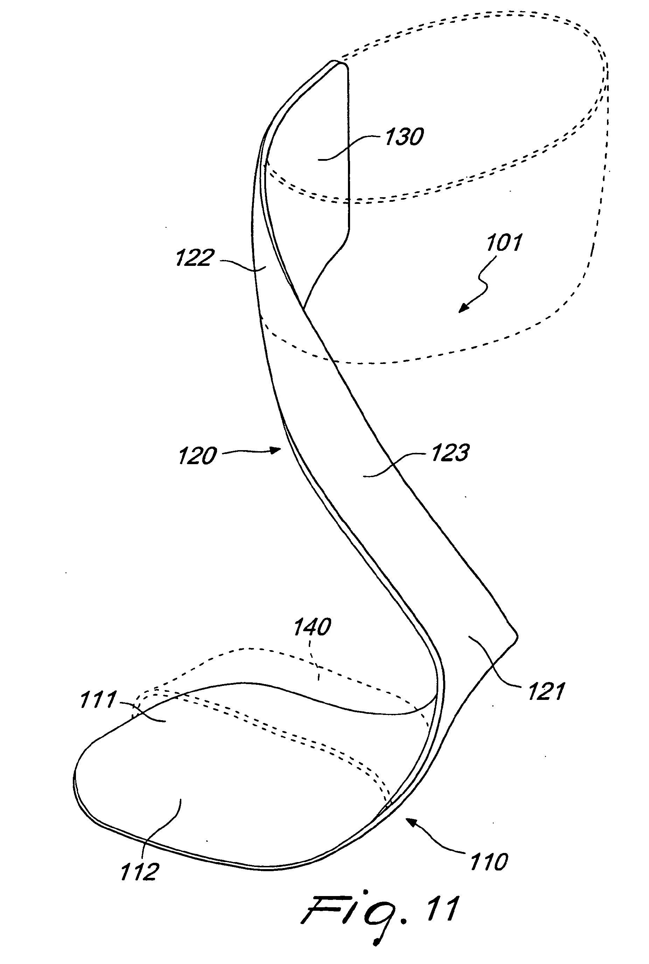

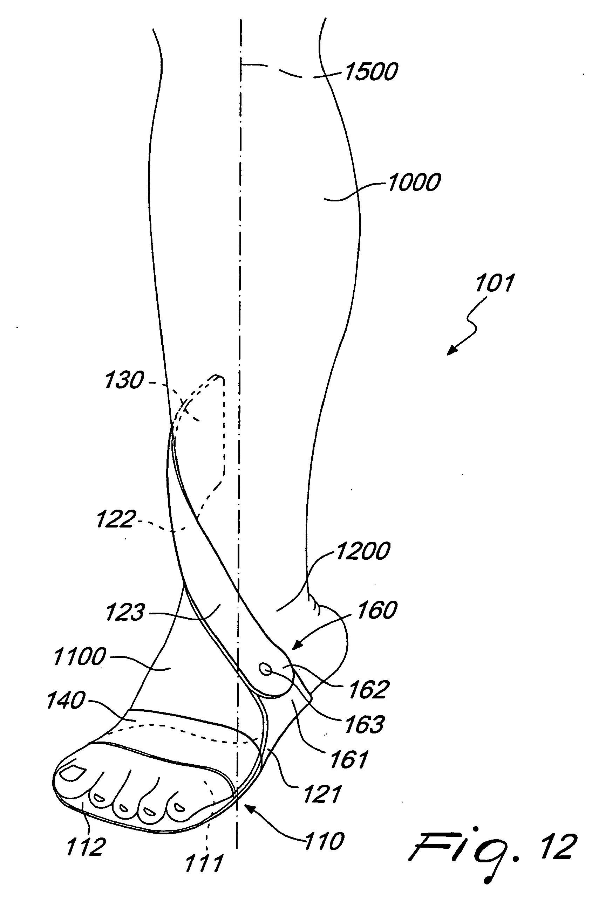

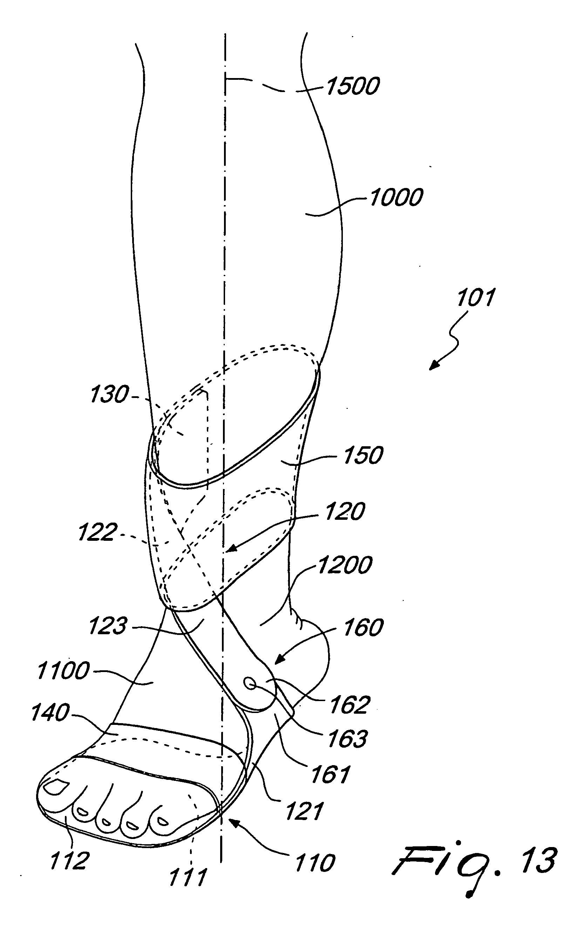

With reference to figures 9 - 13, an orthosis structure, according to a further aspect of the invention, is globally designated by the reference numeral 101.

The orthosis structure 101 comprises a sole-piece 110 which can be associated with the sole of a foot 1100, which is articulated with a leg 1000.

The lateral - or outer - portion of the sole-piece 110 is joined to medial gripping members, which can be engaged with the medial - or inner - zone of the leg 1000.

The medial gripping members, as well as the rest of the sole-piece 110, are suitably made of a composite material of the polymer type. Consequently, they are composed of a matrix which gives them their form and which contains a reinforcement consisting of carbon fibres, Kevlar or other synthetic polymer fibres.

The medial gripping members mainly take the form of a shaped lever 120, which is designed to transmit rigidly to the medial zone of the leg 1000 the supinatory thrust exerted by the foot 1100 on the sole-piece 110.

A strap 140, which will be described further on, has the important function of bearing the other half of the supinatory thrust exerted by the foot.

The terminal portion 121 of the shaped lever 120 is joined to the side portion of the sole-piece 110, while a contact portion 130, which can be engaged with the medial zone of the leg 1000, is formed on its apical portion 122.

It is therefore important to note that the point where the shaped lever 120 is joined to the sole-piece 110, which corresponds essentially to the point of application of the supinatory thrust 2000 exerted by the foot 1100, and the contact portion 130, which corresponds essentially to the point of application of the reaction of the constraint 3000, are situated on opposite sides to each other relative to the longitudinal axis 1500 of the leg 1000.

The shaped lever 120 extends from the sole-piece 110, substantially along the portion associated with the midfoot, and extends so as to pass over the front zone of the leg 1000, without necessarily making contact therewith. The shaped lever 120 rests against the medial zone of the leg 1000 with the contact portion 130.

The shaped lever 120 is inclined forwards, in the antero-posterior plane, at an angle variable substantially between 5° and 15° relative to the perpendicular of the sole-piece 110. It is thus possible to counteract hyperextension of the knee during the putting-down phase.

The shaped lever 120 is also provided with anatomical padding (not shown in the accompanying figures) which lines it internally, improving the comfort during use.

According to the invention, the sole-piece 110 comprises a rigid portion 111 integral with the medial gripping members. The rigid portion 111 can be engaged with the anterior part of the metatarsal zone of the foot 1100 so as to provide a rigid constraint opposing the supinatory thrust exerted by the latter.

The rigid portion 111 is joined laterally to the terminal portion 121 of the shaped lever 120 and is modelled so as to engage with the metatarsal zone, leaving the hindfoot completely free.

A flexible portion 112 is joined as one piece at the front of the rigid portion 111 and is able to return elastically part of the flexural force during the terminal phase of putting down of the foot 1100, allowing, during walking, a more gradual transfer between the putting-down phase, the pushing-off phase and the toe-off phase.

The flexible portion 112 can be engaged with the zone of the foot 1100 substantially delimited by the phalanxes and by the distal metatarsal ends.

The different elastic response between the two portions described may be advantageously obtained by choosing and suitably combining the matrix and the fibres, which form the composite material from which the orthosis structure 101 is made.

The elasticity of the orthosis structure 101 in the antero-posterior plane may be predefined or may be adjustable, namely may be adjusted to the requirements, which may vary over time, of the neurological or orthopaedic patient, and to the lever forces to be opposed. In this latter case, the orthosis structure 101 will be provided with adjustable stiffening means (not shown in the accompanying figures) associated with the points where there is greatest flexing of the shaped lever 120.

The adjustable stiffening means may comprise, for example, a rigid slider, which can be associated horizontally with the central portion 123 of the shaped lever 120.

In order to oppose more effectively the supinatory thrust, the foot 1100 is constrained to the sole-piece 110 by restrain means, which extends from the medial portion of the latter, embraces the dorsal portion of the foot 1100 and reaches the lateral portion of the sole-piece 110.

Namely, the restrain means takes the form of a first strap 140.

The first strap 140 can be associated, in a removable manner, with the single rigid portion 111 only, or with the flexible portion 112 too, so as to engage transversely with the midfoot and forefoot, retaining if necessary also the toes of the foot.

The dynamic response of the medial gripping members may be increased by controlled-movement articulation means which allow a limited and reversible displacement in the antero-posterior plane of said gripping members with respect to the sole-piece 110 so as to favour dorsal flexion of the foot and flexing of the knee. The articulation means also ensure the elastic return into a predefined adjustable initial position when the cause of the stress ceases.

The presence of the articulation means, together with combination of the flexible portion 112 with the rigid portion 111, provide the orthosis structure 101 with maximum rigidity in the frontal plane and elasticity in the antero-posterior plane so as to favour flexing of the knee, ankle and toes.

According to an embodiment of the invention, the articulation means comprises a first resilient hinge 160 formed directly on the shaped lever 120, substantially along the portion which can be associated with the ankle 1200 of the leg 1000 or if necessary consisting of a suitable separate device incorporated in the said shaped lever 120 during manufacture.

The resilient hinge 160 is essentially composed of a first pair of arms 161 and 162 which are pivotally connected together by means of a joining member 163 and are respectively connected to the shaped lever 120 and to the sole-piece 110 so as be more or less parallel to the antero-posterior plane. These arms are constrained by a first spring (not shown in the accompanying figures) which limits the relative movement thereof and tends to bring them back automatically into the initial condition when the stress causing the displacement ceases.

The two arms 161 and 162 are substantially flat and may have substantially discshaped ends which are arranged on top of each other with the possibility of rotation.

The retaining means also comprises an adjustable band 150, which is preferably made, like the first strap 140, of multi-layered fabric, which ensures stability and breathability.

The adjustable band 150 is removably associated with the contact portion 130 alone or with the entire shaped lever 120 so as to engage with the zone of the leg 1000 substantially delimited by the malleolus and by the apical portion of the said contact portion 130.

According to a further embodiment of the invention, not shown in the accompanying figures, the articulation means may consist of a second resilient hinge 120 formed directly on the shaped lever 120, substantially along the central portion 123, or consisting of a suitable separate device incorporated in the said shaped lever 120 during manufacture.

The second resilient hinge comprises a second pair of substantially flat arms which are joined together so as to be slidable and are respectively connected to the terminal portion 121 and to the apical portion 122 of the shaped lever 120 so as to be substantially parallel to the frontal plane and transverse to the antero-posterior plane. These arms are constrained by a suitable restraining device consisting for example of a pawi associated with one of the two arms and a series of corresponding holes formed in the other arm.

In any case, the restraining device is designed to limit the relative movement of the two arms of the second pair and bring them back automatically into the initial condition when the stress which caused the change in their relative position ceases.

While the rigidity of the first arm increases closer to the terminal portion 121 of the shaped lever 120, the rigidity of the second arm is increased by a pair of guides, which slidably receive the first arm.

As a result of this fact, if the first arm is fully housed inside the second arm, the orthoses structure 1 achieves the maximum rigidity envisaged in the antero-posterior plane, while conversely, when the two arms move away from each other, the anteroposterior elasticity increases.

The operating principle of the orthosis structure according to the invention is described below.

The orthosis structure 101 is fitted by associating the sole-piece 110 with the sole of the foot 1100, fixing it with the first strap 140 and resting the contact portion 130 against the medial zone of the leg 1000, fixing it using the second strap 150.

As mentioned, when the foot 1100 exerts its supinatory thrust on the rigid portion 111 of the sole-piece 110, the latter, by means of the shaped lever 120, transmits it rigidly to the contact portion 130, which encounters a physical constraint in the medial zone of the leg 1000.

In practice, this results in the formation of a line, rigid on the frontal plane, which starts from the medial portion of the sole of the forefoot, comprising the distal portion of the metatarsus, extends to the lateral edge of the sole of the foot 1100 and returns again into the medial position, passing diagonally over the instep. The line embraces, without necessarily making contact, the frontal zone of the lowest portion of the leg 1000 and then makes contact with the medial surface of the same leg 1000, at least above the tibial malleolus, where the supinatory lever force is discharged, encountering a solid constraint.

While the lateral side of the rigid plantar bears the downward rotational thrust of the lateral side of the forefoot, the strap 140 has the important function of bearing the other half, upward rotational thrust of the medial side of the forefoot.

It must be pointed out that, during walking, the flexible portion 112 allows a more gradual transfer from the putting-down phase to the pushing-off phase of the foot 1100. If necessary, controlled-movement articulation means may also be used, allowing a limited and reversible displacement of the medial gripping members with respect to the sole-piece 110, increasing further the rigidity in the frontal plane and the elasticity in the antero-posterior plane so as to favour the correct dynamics of the walking movement.

The structure according to the present invention is resilient on its sagittal plane, i.e. on the median longitudinal plane of the leg and foot, while being substantially stiff in its frontal, or transversal plane. Such constructive features may be achieved by suitably orienting the fibers and sheets that constitute the carbon fiber structure.

It will be appreciated that the structure according to the present invention substantially lack a heel portion of the sole-piece, 2 or 110, and substantially lack any rear and inner portion of the shaped lever 11, 120 extending beyond the plate 12, or contact portion 130. In other words, the shaped lever 11, 120 ends at the medial side of the calf without extending back in the posterior side of the calf, because there is no need of tightly binding the calf in the sagittal plane.

The lack of a back and inner portion of the shaped lever is extremely advantageous and makes the orthosis structure according to the present invention very easy to wear.

It has been shown in practice how the orthosis structure according to the invention fully achieves the intended aims and objects.

The orthosis structure according to the invention is particularly effective in counteracting the deformities in valgus-pronation or the spasticity in varus-supination of the foot, ensuring a good stability of the ankle and the foot and at the same time a more dynamic and functional walking movement.

Also, the orthosis structure according to the invention controls the foot drop during the swinging phase and ensures a more stable walking movement.

The orthosis structure according to the invention is lightweight and comfortable and also aesthetically pleasing.

The orthosis structure according to the invention is also designed to oppose the supination movement of the foot.

In fact, the orthosis structure according to the invention is able to provide a rigid constraint opposing the supinatory thrust and to support the foot, offering maximum rigidity in the frontal plane, as well as favour the correct dynamics of the gait, owing to its elasticity in the sagittal plane.

The orthosis structure according to the invention helps preventing the hyperextension of the knee and is useful for neurological patients, in order to counteract the unwanted supinatory movement, which is frequently observed as a pathological reflex in pareses due to central neurological lesion, as well as in orthopaedic patients as a means for preventing recurrent twisting of the ankle and instep injuries.

Also, the orthosis structure according to the invention may be used with all commercially available footwear, without having to modify the footwear after purchase.

Also, being slim and lightweight, it is easy and convenient to use, as well as being cooler during the summer months and aesthetically more pleasing in appearance, thereby reducing the embarrassment associated with wearing it together with sandals and shorts or skirts.

In practice, the materials used, provided they are compatible with the specfic use, as well as the related forms and dimensions may be of any nature depending on the requirements and the state of the art.

This application claims the priority of Italian Patent Applications No. VI2008A000110, filed on May 15, 2008 and No. VI2009A000087, filed on April 22, 2009, the subject matter of which is incorporated herein by reference.

## Claims

1. Ankle foot orthosis for counteracting the supination of the foot,
comprising a sole-piece (2; 110) which can be associated with the sole of a foot, and medial gripping means (10) which are joined to said sole-piece and engage the leg, the point where said medial gripping means are joined to said sole-piece being on the lateral edge of the sole-piece,
the point where said medial gripping means are joined to said sole-piece is situated on opposite sides to the portion where said medial gripping means are engaged to said leg, relative to the longitudinal axis of said leg;
said medial gripping means comprising a shaped lever (11; 120) which is joined to the lateral portion of said sole-piece (2; 110) and can be engaged with the medial zone of said leg by means of a contact portion (12; 130), said shaped lever (11; 120) being able to transmit rigidly to the medial zone of said leg the inversion thrust exerted by said foot on the sole-piece (2; 110),
in which the shaped lever (11; 120) extends from the sole-piece (2; 110) substantially along the portion associated with the midfoot and extends so as to pass over the front pre-tibial zone of the leg (60), without necessary contact with it,
said sole-piece (110) comprises:
- at least one rigid portion (111) integral with said medial gripping means, said rigid portion being able to be engaged with the distal metatarsal zone of said foot so as to provide a rigid constraint opposing the downward rotational thrust of the lateral side of the forefoot; and
- a flexible portion (112) joined as one piece to said rigid portion (111), said flexible portion being able to be engaged with the zone of said foot substantially delimited by the phalanxes and by the distal metatarsal ends so as to return part of the flexural force during the terminal phase of putting-down of said foot,
the ankle foot orthosis further comprises a restrain means associated with said sole-piece,
in which said restrain means are suitable for counteracting the tendency of the medial portion of the foot to raise by constraining said foot to said sole-piece, said restrain means extending from the medial portion of the sole-piece, embracing the dorsal portion of the foot (50; 1100) and reaching the lateral portion of the sole-piece, whereby being suitable for bearing the upward rotational thrust of the medial side of the forefoot.

2. Ankle foot orthosis according to claim 1, wherein a terminal portion of said shaped lever is joined laterally to said rigid portion (111) of said sole-piece and an apical portion of said shaped lever (120) is able to be engaged with the medial zone of said leg, said shaped lever (120) transmitting rigidly to the medial zone of said leg the supinatory thrust exerted by said foot on said sole-piece (110).

3. Ankle foot orthosis according to claim 2, wherein said medial gripping means comprise at least one contact portion formed substantially on said apical portion of said shaped lever (120).

4. Ankle foot orthosis according to one or more of the preceding claims, wherein said shaped lever (11; 120) comprises a plate (12; 130) defined in the region of said engageable portion, said plate embracing the medial side portion of said leg.

5. Ankle foot orthosis according to one or more of the preceding claims, wherein said shaped lever (11; 120) comprises a rigid core which extends inside said sole-piece (2; 110) and/or **characterized in that** said sole-piece comprises a non-slip sole associated underneath.

6. Ankle foot orthosis according to one or more of the preceding claims, wherein said sole-piece (2) comprises at least one flange (13) which extends medially and preferably **characterized in that** said restrain means comprises at least one strap (21) which can be removably associated with said flange (13) and said shaped lever (11).

7. Ankle foot orthosis, according to one or more of the preceding claims, wherein it comprises a retaining means which can be removably associated with said medial gripping members, said retaining means constraining said leg to said contact portion.

8. Ankle foot orthosis, according to one or more of the preceding claims, wherein said medial gripping means comprise controlled-movement articulation means, said articulation means allowing a limited and reversible displacement in the sagittal plane of said medial gripping means with respect to said sole-piece, said displacement favouring flexing of the ankle, said articulation means also causing the elastic return into the initial condition when the cause of the stress ceases.

9. Ankle foot orthosis, according to claim 8, wherein said articulation means comprise a first resilient hinge (160) formed substantially along the side portion of said shaped lever (120) which can be associated with the ankle, said side portion being substantially parallel to the antero-posterior plane.

10. Ankle foot orthosis, according to claim 9, wherein said first resilient hinge (160) comprises a first pair of arms (161, 162) arranged on top of each other and coupled rotably together, the arms of said first pair being constrained by a first spring and being respectively connected to said shaped lever (120) and to said rigid portion.

11. Ankle foot orthosis, according to one or more of claims 8-10, wherein said articulation means comprise a second resilient hinge formed substantially along the central portion (123) of said shaped lever (120) which can be associated with said ankle, said central portion being substantially parallel to the frontal plane and transverse to said antero-posterior plane.

12. Ankle foot orthosis, according to one of claims 8-11, wherein said first sliding hinge comprises a second pair of arms arranged on top of each other and coupled together telescopically, the arms of said second pair being constrained by a restraining device and being respectively connected to said apical portion and to said terminal portion of said shaped lever

13. Ankle foot orthosis, according to one or more of the preceding claims, wherein it comprises adjustable stiffening means which are associated with the points where there is greatest flexing of said orthosis structure in which said adjustable stiffening means preferably comprise at least one rigid slider which can be associated with said central portion of said shaped lever which can be associated with said ankle, said central portion being transverse to the antero-posterior plane.

14. Ankle foot orthosis, according to one or more of the preceding claims, in which the structure of the orthosis forms a line, rigid on the frontal plane, which starts from the medial portion of the sole of the forefoot, comprising the distal portion of the metatarsus, said forefoot portion being constrained to said sole-piece by said retain means, extends to the lateral edge of the sole of the foot and returns again into the medial side of the leg, passing diagonally over the instep, the line embracing the frontal zone of the lowest portion of the leg and then making contact with the medial surface of the same leg where the supinatory lever force is discharged encountering a solid constrain, and preferably in which a contact portion (130) of said shaped lever (120) is a portion located longitudinally with said shaped lever (120).

15. Ankle foot orthosis, according to claim 1, in which the sole-piece (2; 110) is suitable for being associated with the sole of a foot leaving the hindfoot free.

16. Ankle foot orthosis, according to claim 1, in which the restraining means have a lateral end joined to said medial gripping means (10).

## Patentansprüche

1. Fußgelenkorthese zur Unterdrückung der Aufwärtsdrehung des Fußes,
umfassend ein Sohlenstück (2; 110), das mit einer Fußsohle verbunden werden kann, sowie ein mediales Greifmittel (10), das mit dem Sohlenstück zusammengefügt ist und das Bein umgreift, wobei der Punkt, an dem das mediale Greifmittel mit dem Sohlenstück zusammengefügt ist, an der lateralen Kante des Sohlenstücks liegt,
wobei sich der Punkt, an dem das mediale Greifmittel mit dem Sohlenstück zusammengefügt ist, relativ zur Längsachse des Beins, an der Seite gegenüber dem Abschnitt befindet, an dem das mediale Greifmittel das Bein umgreift;
wobei das Greifmittel einen geformten Hebel (11; 120) umfasst, der mit dem lateralen Abschnitt des Sohlenstücks (2; 110) zusammengefügt ist und den medialen Bereich des Beins mittels eines Berührungsabschnitts (12; 130) umgreifen kann, wobei der geformte Hebel (11; 120) dazu fähig ist, an den medialen Bereich des Beins den vom Fuß auf das Sohlenstück (2; 110) ausgeübten Umkehrdruck starr zu übertragen,
wobei sich der geformte Hebel (11; 120) vom Sohlenstück (2; 110) im Wesentlichen entlang dem Abschnitt erstreckt, der mit dem Mittelfuß verbunden ist und sich derart erstreckt, dass er über den Prätibialbereich des Beins (60) verläuft, ohne ihn notwendigerweise zu berühren,
wobei das Sohlenstück (110) Folgendes umfasst:
- mindestens einen starren Abschnitt (111), der mit dem medialen Greifmittel einstückig verbunden ist, wobei der starre Abschnitt dazu fähig ist, derart den distalen Metatarsalbereich des Fußes zu umgreifen, dass ein starres Verbinden bereitgestellt ist, das dem nach unten gerichteten Rotationsdruck der lateralen Seite des Vorderfußes entgegenwirkt; und
- einen flexiblen Abschnitt (112), der einstückig mit dem starren Abschnitt (111) zusammengefügt ist, wobei der flexible Abschnitt dazu fähig ist, den Bereich des Fußes, der im Wesentlichen von den Phalangen und den distalen Metatarsalenden begrenzt wird, zu umgreifen, sodass er einen Teil der Biegekraft während der Endphase des Absetzens des Fußes zurückgibt,
wobei die Fußgelenkorthese ferner ein Zurückhaltemittel umfasst, das mit dem Sohlenstück verbunden ist,
wobei das Zurückhaltemittel zur Unterdrückung der Tendenz des medialen Abschnitts des Fußes zum Anheben durch ein Verbinden des Fußes mit dem Sohlenstück geeignet ist, wobei sich das Zurückhaltemittel vom medialen Abschnitt des Sohlenstücks erstreckt, den dorsalen Abschnitt des Fußes (50; 1100) umschließt und den lateralen Abschnitt des Sohlenstücks erreicht, wobei es dazu geeignet ist, dem nach oben gerichteten Rotationsdruck der medialen Seite des Vorderfußes standzuhalten.

2. Fußgelenkorthese nach Anspruch 1, wobei ein Endabschnitt des geformten Hebels lateral mit dem starren Abschnitt (111) des Sohlenstücks zusammengefügt ist und ein apikaler Abschnitt des geformten Hebels (120) dazu fähig ist, den medialen Bereich des Beins zu umgreifen, wobei der geformte Hebel (120) starr an den medialen Bereich des Beins den Aufwärtsdruck überträgt, der vom Fuß auf das Sohlenstück (110) ausgeübt wird.

3. Fußgelenkorthese nach Anspruch 2, wobei das mediale Greifmittel mindestens einen Berührungsabschnitt umfasst, der im Wesentlichen auf dem apikalen Abschnitt des geformten Hebels (120) ausgebildet ist.

4. Fußgelenkorthese nach einem oder mehreren der vorangehenden Ansprüche, wobei der geformte Hebel (11; 120) eine Platte (12; 130) umfasst, die im Gebiet des umgreifbaren Abschnitts ausgebildet ist, wobei die Platte den medialen Seitenabschnitt des Beins umschließt.

5. Fußgelenkorthese nach einem oder mehreren der vorangehenden Ansprüche, wobei der geformte Hebel (11; 120) einen starren Kern umfasst, der sich innerhalb des Sohlenstücks (2; 110) erstreckt und/oder **dadurch gekennzeichnet ist, dass** das Sohlenstück eine rutschfeste Sohle umfasst, die darunter mit ihm verbunden ist.

6. Fußgelenkorthese nach einem oder mehreren der vorangehenden Ansprüche, wobei das Sohlenstück (2) mindestens einen Gurt (13) aufweist, der sich medial erstreckt und vorzugsweise **dadurch gekennzeichnet ist, dass** das Zurückhaltemittel mindestens einen Riemen (21) umfasst, der entfernbar mit dem Gurt (13) und dem geformten Hebel (11) verbunden werden kann.

7. Fußgelenkorthese nach einem oder mehreren der vorangehenden Ansprüche, wobei sie ein Haltemittel umfasst, das entfernbar mit den medialen Greifmitteln verbunden werden kann, wobei das Haltemittel das Bein mit dem Berührungsabschnitt verbindet.

8. Fußgelenkorthese nach einem oder mehreren der vorangehenden Ansprüche, wobei das mediale Greifmittel ein Mittel zur kontrollierten Artikulationsbewegung umfasst, wobei das Artikulationsmittel eine begrenzte und reversible Verschiebung in der Sagittalebene des medialen Greifmittels in Bezug auf das Sohlenstück gestattet, wobei die Verschiebung eine Beugung des Fußgelenks begünstigt, wobei das Artikulationsmittel zudem das elastische Zurückkehren in den Anfangszustand verursacht, wenn die Ursache für die Belastung aufhört.

9. Fußgelenkorthese nach Anspruch 8, wobei das Artikulationsmittel ein erstes nachgiebiges Gelenkscharnier (160) umfasst, das im Wesentlichen entlang dem Seitenabschnitt des geformten Hebels (120) ausgebildet ist, der mit dem Fußgelenk verbunden werden kann, wobei der Seitenabschnitt im Wesentlichen parallel zur anteroposterioren Ebene ist.

10. Fußgelenkorthese nach Anspruch 9, wobei das erste nachgiebige Gelenkscharnier (160) ein erstes Paar Arme (161, 162) umfasst, die übereinander angeordnet und rotierbar aneinandergekoppelt sind, wobei die Arme des ersten Paares durch eine erste Feder miteinander verbunden sind und jeweils an den geformten Hebel (120) und den starren Abschnitt anschließen.

11. Fußgelenkorthese nach einem oder mehreren der vorangehenden Ansprüche 8 bis 10, wobei das Artikulationsmittel ein zweites nachgiebiges Gelenkscharnier umfasst, das im Wesentlichen entlang dem zentralen Abschnitt (123) des geformten Hebels (120) ausgebildet ist, der mit dem Fußgelenk verbunden werden kann, wobei der zentrale Abschnitt im Wesentlichen parallel zur Frontalebene und transversal zur anteroposterioren Ebene ist.

12. Fußgelenkorthese nach einem oder mehreren der vorangehenden Ansprüche 8 bis 11, wobei das erste gleitende Gelenkscharnier ein zweites Paar Arme umfasst, die übereinander angeordnet und teleskopartig aneinandergekoppelt sind, wobei die Arme des zweiten Paares durch eine zurückhaltende Vorrichtung miteinander verbunden sind und jeweils an den apikalen Abschnitt und den Endabschnitt des geformten Hebels anschließen.

13. Fußgelenkorthese nach einem oder mehreren der vorangehenden Ansprüche, wobei sie ein anpassbares Versteifungsmittel umfasst, das mit den Punkten verbunden ist, an denen die größte Beugung der Ortheseanordnung vorliegt, wobei das anpassbare Versteifungsmittel vorzugsweise mindestens ein starres Gleitstück umfasst, das mit dem zentralen Abschnitt des geformten Hebels verbunden werden kann, der mit dem Fußgelenk verbunden werden kann, wobei der zentrale Abschnitt transversal zur anteroposterioren Ebene ist.

14. Fußgelenkorthese nach einem oder mehreren der vorangehenden Ansprüche, wobei die Anordnung der Orthese eine Linie bildet, die starr in der Frontalebene liegt, die am medialen Abschnitt der Sohle des Vorderfußes beginnt, umfassend den distalen Abschnitt des Metatarsus, wobei der Vorderfußabschnitt mit dem Sohlenstück durch das Haltemittel verbunden wird, das sich zur lateralen Kante der Fußsohle erstreckt und wieder in die mediale Seite des Beins zurückkehrt, wobei sie diagonal über den Spann verläuft, wobei die Linie den frontalen Bereich des untersten Abschnitts des Beins umschließt und anschließend die mediale Oberfläche des Beins berührt, wo die Aufwärtshebelkraft beim Auftreffen auf eine solide Verbindung aufgelöst wird und wobei vorzugsweise ein Berührungsabschnitt (130) des geformten Hebels (120) ein Abschnitt ist, der sich longitudinal zum geformten Hebel (120) befindet.

15. Fußgelenkorthese nach Anspruch 1, wobei das Sohlenstück (2; 110) dazu geeignet ist, mit der Sohle eines Fußes verbunden zu werden, wobei der Hinterfuß freigelassen wird.

16. Fußgelenkorthese nach Anspruch 1, wobei das Zurückhaltemittel ein laterales Ende aufweist, das mit dem medialen Greifmittel (10) zusammengefügt ist.

## Revendications

1. Orthèse de la cheville destinée à contrer la supination du pied,
comprenant une pièce de semelle (2 ; 110) qui peut être associée à la plante d'un pied, et des moyens de saisie médiale (10) qui sont reliés à ladite pièce de semelle et engagent la jambe, le point auquel lesdits moyens de saisie médiale sont reliés à ladite pièce de semelle, se situant sur le bord latéral de la pièce de semelle, le point auquel lesdits moyens de saisie médiale sont reliés à ladite pièce de semelle, se situe sur des côtés opposés à la partie où lesdits moyens de saisie médiale sont engagés sur ladite jambe, par rapport à l'axe longitudinal de ladite jambe ;
lesdits moyens de saisie médiale comprenant un levier façonné (11 ; 120) qui est relié à la partie latérale de ladite pièce de semelle (2 ; 110) et peut s'engager avec la région médiale de ladite jambe par le biais d'une partie de contact
(12; 130), ledit levier façonné (11 ; 120) pouvant transmettre, rigidement à la région médiale de ladite jambe, la
poussée d'inversion exercée par ledit pied sur la pièce de semelle (2 ; 110),
dans laquelle le levier façonné (11 ; 120) se développe depuis la pièce de semelle (2 ; 110), essentiellement le long de la partie associée au médio-pied, et continue de sorte à passer sur la région pré-tibiale antérieure de la jambe (60), sans nécessairement la toucher,
ladite pièce de semelle (110) comprend :
- au moins une partie rigide (111) solidaire desdits moyens de saisie médiale, ladite partie rigide pouvant s'engager avec la région métatarsienne distale dudit pied, de sorte à exercer une contrainte rigide s'opposant à la poussée rotative descendante du côté latéral de l'avant-pied ; et
- une partie flexible (112) reliée d'un seul tenant à ladite partie rigide (111), ladite partie flexible pouvant s'engager avec la région dudit pied, essentiellement délimitée par les phalanges et par les extrémités distales des métatarsiens, de sorte à restituer une partie de la force de flexion pendant la phase terminale de pose dudit pied,
l'orthèse de la cheville comprend également des moyens de retenue associés à ladite pièce de semelle,
dans laquelle lesdits moyens de retenue peuvent contrer la tendance de la partie médiale du pied de se lever en contraignant ledit pied à ladite pièce de semelle, lesdits moyens de retenue se développant depuis la partie médiale de la pièce de semelle, englobant la partie dorsale du pied (50 ; 1100) et atteignant la partie latérale de la pièce de semelle, étant donc en mesure de supporter la poussée rotative ascendante du côté médial de l'avant-pied.

2. Orthèse de la cheville selon la revendication 1, dans laquelle une partie terminale dudit levier façonné est reliée latéralement à ladite partie rigide (111) de ladite pièce de semelle, et une partie apicale dudit levier façonné (120) peut s'engager avec la région médiale de ladite jambe, ledit levier façonné (120) transmettant, rigidement à la région médiale de ladite jambe, la poussée supinatrice exercée par ledit pied sur ladite pièce de semelle (110).

3. Orthèse de la cheville selon la revendication 2, dans laquelle lesdits moyens de saisie médiale comprennent au moins une partie de contact, formée essentiellement sur ladite partie apicale dudit levier façonné (120).

4. Orthèse de la cheville selon l'une ou plusieurs des revendications précédentes, dans laquelle ledit levier façonné (11 ; 120) comprend une plaque (12 ; 130) située dans la région de ladite partie engageable, ladite plaque englobant la partie du côté médial de ladite jambe.

5. Orthèse de la cheville selon l'une ou plusieurs des revendications précédentes, dans laquelle ledit levier façonné (11 ; 120) comprend une âme rigide qui évolue à l'intérieur de ladite pièce de semelle (2 ; 110), et/ou **caractérisée en ce que** ladite pièce de semelle comprend une semelle antidérapante associée au-dessous.

6. Orthèse de la cheville selon l'une ou plusieurs des revendications précédentes, dans laquelle ladite pièce de semelle (2) comprend au moins une bride (13) qui évolue de façon médiale et, de préférence, **caractérisée en ce que** lesdits moyens de retenue comprennent au moins une sangle (21), qui peut être associée de manière amovible à ladite bride (13) et audit levier façonné (11).

7. Orthèse de la cheville selon l'une ou plusieurs des revendications précédentes, qui comprend des moyens de retenue pouvant être associés de manière amovible auxdits organes de saisie médiale, lesdits moyens de retenue contraignant ladite jambe à ladite partie de contact.

8. Orthèse de la cheville selon l'une ou plusieurs des revendications précédentes, dans laquelle lesdits moyens de saisie médiale comprennent des moyens d'articulation à mouvement contrôlé, lesdits moyens d'articulation permettant un déplacement limité et réversible dans le plan sagittal desdits moyens de saisie médiale par rapport à ladite pièce de semelle, ledit déplacement facilitant la flexion de la cheville, lesdits moyens d'articulation entraînant également le retour élastique dans la condition initiale quand cesse la cause de la tension.

9. Orthèse de la cheville selon la revendication 8, dans laquelle lesdits moyens d'articulation comprennent une première charnière élastique (160), formée essentiellement le long de la partie latérale dudit levier façonné (120), qui peut être associée à la cheville, ladite partie latérale étant essentiellement parallèle au plan antéropostérieur.

10. Orthèse de la cheville selon la revendication 9, dans laquelle ladite première charnière élastique (160) comprend une première paire de bras (161, 162), mis au-dessus l'un de l'autre et accouplés de manière rotative, les bras de ladite première paire étant contraints par un premier ressort et étant raccordés respectivement audit levier façonné (120) et à ladite partie rigide.

11. Orthèse de la cheville selon l'une ou plusieurs des revendications 8-10, dans laquelle lesdits moyens d'articulation comprennent une seconde charnière élastique, formée essentiellement le long de la partie centrale (123) dudit levier façonné (120), qui peut être associée à ladite cheville, ladite partie centrale étant essentiellement parallèle au plan antérieur et transversale audit plan antéropostérieur.

12. Orthèse de la cheville selon l'une des revendications 8-10, dans laquelle ladite première charnière coulissante comprend une seconde paire de bras, mis au-dessus l'un de l'autre et accouplés de manière télescopique, les bras de ladite seconde paire étant contraints par un dispositif de retenue et étant raccordés respectivement à ladite partie apicale et à ladite partie terminale dudit levier façonné.

13. Orthèse de la cheville selon l'une ou plusieurs des revendications précédentes, qui comprend des moyens de raidissement réglables associés aux points où la structure de ladite orthèse présente la plus grande flexion, dans laquelle lesdits moyens de raidissement réglables comprennent de préférence au moins un coulisseau rigide pouvant être associé à ladite partie centrale dudit levier façonné qui peut être associé à ladite cheville, ladite partie centrale étant transversale au plan antéropostérieur.

14. Orthèse de la cheville selon l'une ou plusieurs des revendications précédentes, dans laquelle la structure de l'orthèse forme une ligne, rigide sur le plan antérieur, qui part de la partie médiale de la plante de l'avant-pied, comprenant la partie distale du métatarse, ladite partie de l'avant-pied étant contrainte à ladite pièce de semelle par lesdits moyens de retenue, se poursuit vers le bord latéral de la plante du pied et retourne dans le côté médial de la jambe, passant diagonalement sur le cou-de-pied, la ligne englobant la région antérieure de la partie inférieure de la jambe et touchant ensuite la surface médiale de ladite jambe où la force de levier supinatrice est dégagée, rencontrant une contrainte solide, et de préférence dans laquelle une partie de contact (130) dudit levier façonné (120) est une partie située longitudinalement audit levier façonné (120).

15. Orthèse de la cheville selon la revendication 1, dans laquelle la pièce de semelle (2 ; 110) peut être associée à la plante d'un pied, laissant l'arrière-pied libre.

16. Orthèse de la cheville selon la revendication 1, dans laquelle les moyens de retenue présentent une extrémité latérale reliée auxdits moyens de saisie médiale (10).
